Europäisches Patentamt

European Patent Office

Office européen des brevets

(11) Publication number: **0 098 442**
**B1**

# (12) EUROPEAN PATENT SPECIFICATION

(45) Date of publication of patent specification: **11.03.87**

(51) Int. Cl.⁴: **A 61 B 3/04**

(21) Application number: **83106014.0**

(22) Date of filing: **20.06.83**

(54) A motor-driven subjective phorometer.

(30) Priority: **25.06.82 JP 109451/82**

(43) Date of publication of application:
**18.01.84 Bulletin 84/03**

(45) Publication of the grant of the patent:
**11.03.87 Bulletin 87/11**

(84) Designated Contracting States:
**DE GB**

(56) References cited:
**EP-A-0 044 770**
**EP-A-0 045 897**
**DE-A-2 901 459**
**DE-A-3 037 466**
**US-A-3 498 699**
**US-A-3 791 719**

(73) Proprietor: **Hoya Lens Corporation**
**25, Kowada Itsukaichi-machi Nishitama-gun**
**Tokyo (JP)**

(72) Inventor: **Hayao, Akaba**
**2-18-12 Haijima-cho**
**Akishima-shi Tokyo (JP)**
Inventor: **Takeshi, Yamada**
**Sakae-cho No. 508 1-6-19 Sakae-cho, Hamura-machi**
**Nishitama-gun Tokyo (JP)**
Inventor: **Masahiro, Jinbo**
**423-42 Sunagawa-cho**
**Tachikawa-shi Tokyo (JP)**

(74) Representative: **Blumbach Weser Bergen**
**Kramer Zwirner Hoffmann Patentanwälte**
**Radeckestrasse 43**
**D-8000 München 60 (DE)**

Courier Press, Leamington Spa, England.

## Description

This invention relates to a motor-driven subjective phorometer.

In prior subjective phorometers, rotary motion of each of their plates which carry respective series of lenses (hereinafter referred to as lens plates) is produced by means of a knob connected directly or by means of gearing to the lens plates. Raising and lowering one of the eye heads, changing the pupillary distance, and causing convergence of the left-eye head and the right-eye head are also effected by knobs connected thereto by gearing, so that the knobs are mounted separately at different spaced locations, and the data measured therewith must be read off on separate scales and display boards.

A motor-driven subjective phorometer according to the first portion of Claim 1 is disclosed in EP—A—0,045,897. This known phorometer employs fine stepping motors driven by control and drive signals electronically generated by a computer system. The document does not disclose details on the drive system for moving the lens plates. The mounting means for the lens plates are manually driven in this prior art.

An object of this invention is to provide a motor-driven subjective phorometer in which all the measuring means can be actuated simply by a group of control switches or keys gathered in a central panel, and all the data measured can be displayed digitally in a monitor and printed as required, while data possessed previously or obtained from other instruments such as an objective phorometer and the like can be inputted thereto simply by the group of control switches or keys.

This object is achieved with a motor-driven subjective phorometer as claimed in Claim 1.

An embodiment of the invention will now be described by way of example with reference to the accompanying drawings in which:

Figures 1A and B are side views of a means for actuating two spherical lens plates, and a means for actuating an additional plate in the right-eye head of a subjective phorometer of this invention, Figure 1A showing the means for actuating a spherical lens plate including a series of spherical lenses each having a different spherical power, varying in small dioptral steps, and Figure 1B showing the means for actuating a spherical lens plate including a series of spherical lenses each having a different spherical power, varying in large dioptral steps;

Figures 2A and B are side views of a means for actuating two toric lens plates of the right-eye head of the subjective phorometer of this invention, Figure 2A showing the means for actuating a toric lens plate including a series of toric lenses each having a different cylindrical power, varying in small dioptral steps, and means for actuating a toric lens plate including a series of toric lenses each having a different cylindrical power, varying in large dioptral steps, and Figure 2B showing the means for actuating all the cylinder axes of the toric lenses of the two toric lens plates;

Figure 3 is a front view of a means for convergence of two plates for the right and left eyes, each plate having all the spherical and toric lens plates mounted thereon, a means for raising or lowering one eye head, and a means for adjusting pupillary distance, of the subjective phorometer;

Figure 4 is a block diagram of the subjective phorometer; and

Figure 5 is a plan view of a control key or switch panel of the subjective phorometer.

The subjective phorometer may be a desk-type apparatus in which all the measuring elements are set on the desk plate or desk top and all the procedures for measuring vision data of eyes are carried out by control keys or switches on the desk, and all the data measured are displayed digitally on a monitor, and printed out as required.

The elements of the phorometer for measuring vision data of eyes such as right-eye and left-eye heads each consisting of two spherical lens plates, two toric lens plates, and an additional plate, an element for raising and lowering the one eye head, an element for convergence of the left-eye head and the right-eye head, and an element for adjusting pupillary distance, and so on, are conventional, but the means for actuating those elements and for displaying the data measured are different from the conventional arrangements. The right-eye head and left-eye head are symmetrical relative to each other, so reference will be made to only one eye head hereinafter.

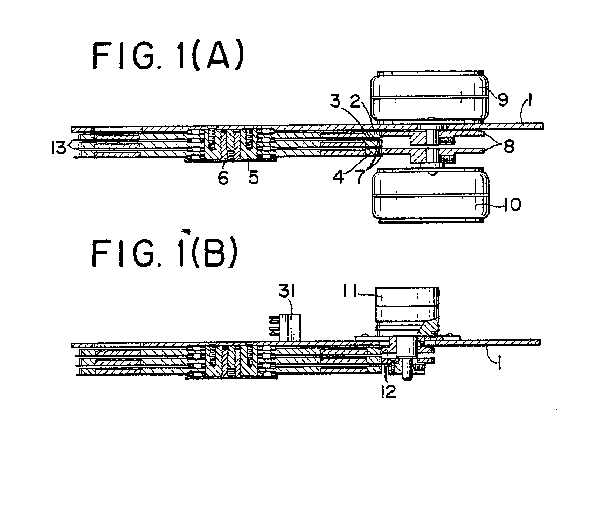

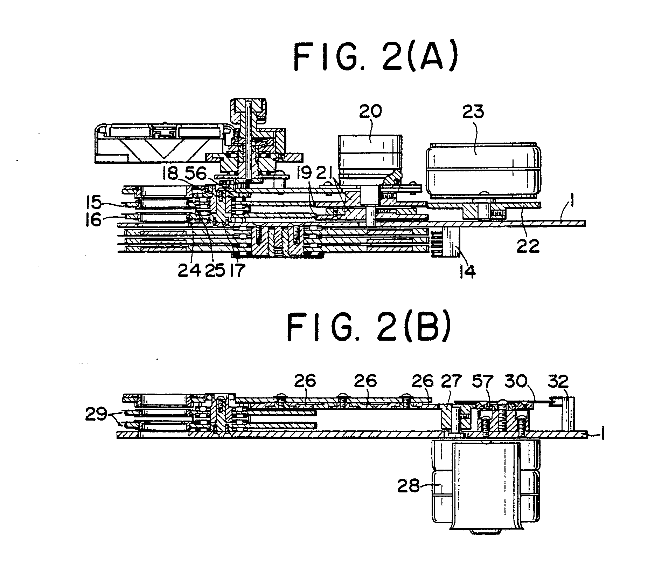

Figure 1 and Figure 2 are side views of means for driving the elements of the right-eye head. In Figure 1A, 1 is a setting plate. A lens plate 2 having a circular series of spherical lenses having different spherical powers, varying in 0.25 diopter steps, a lens plate 3 having a circular series of spherical lenses having different spherical powers, varying in 3.00 diopter steps, and an additional plate 4 having lenses necessary for measuring various kinds of vision data are respectively mounted to a mounting shaft or spindle 5 of the setting plate 1, and are prevented from becoming detached from the shaft 5 by a setting screw 6.

Each plate 2, 3 and 4 is rotatable around the shaft 5, and engages a respective gear 7 at its circumference; the gear 7 of plate 2 engages with the gear 8 fixed to the shaft of a pulse motor 9, and the gear 7 of the additional plate 4 engages with the gear 8 fixed to the shaft of a pulse motor 10.

Figure 1B is a side view of an electro-magnetic clutch assembly for rotating the lens plate 3 as required, in which 11 is an electro-magnetic clutch having a double shaft mechanism, in which the gear 12 fixed to the shaft of the clutch 11 engages with the gear 7 of the lens plate 2 and the gear 7 of the lens plate 3.

Each plate 2, 3 and 4 has, at a position on its circumference, a shielding plate 13 which serves to detect the zero position of each plate by cooperation with a respective optical sensor 14 (shown in Fig. 2A) which is fixed to the setting plate 1, in association with each shielding plate 13.

Figure 2A is a side view of a means for driving two toric lens plates, and Figure 2B is a side view of a means for driving the cylinder axes of all the toric lenses of the two toric lens plates. A lens plate 15 is a toric lens plate having a circular series of five toric lenses each having a different cylindrical power, in 0.25 diopter steps. A lens plate 16 is a toric lens plate having a circular series of toric lenses each having a different cylindrical power, in 1.25 diopter steps. Plates 15 and 16 are respectively rotatably fitted to a shaft 17 and gears 25, both of which are fixed to the setting plate 1, being prevented from becoming detached by a setting screw 18, and gears 56. Lens plates 15 and 16 each engage at their circumference with a gear 19 which engages with gear 21 attached to an electro-magnetic clutch 20 which has a double shaft mechanism; the gear 21 also engages with a gear 22 attached to the shaft of a pulse motor 23.

Each lens in the series of toric lenses arranged in the lens plates 15 and 16 is set in a ring 24 which has a gear at the circumference, and the gear of each ring engages with one central gear 25 which engages with gear 56 and a connecting gear means 26 which engages with a gear 27 attached to a pulse motor 28.

Each of plates 15 and 16 has, at its circumference, a shielding plate 29 which serves to detect the zero position of each plate 15 and 16, by operation of an optical sensor 31 shown in Figure 1B and attached to the setting plate 1.

Each plate 15 and 16 also has, at a gear 57 which engages with gear 27, a shielding plate 30 which serves to detect the zero position of the cylindrical axes of the toric lenses, by means of an optical sensor 32 attached to setting plate 1.

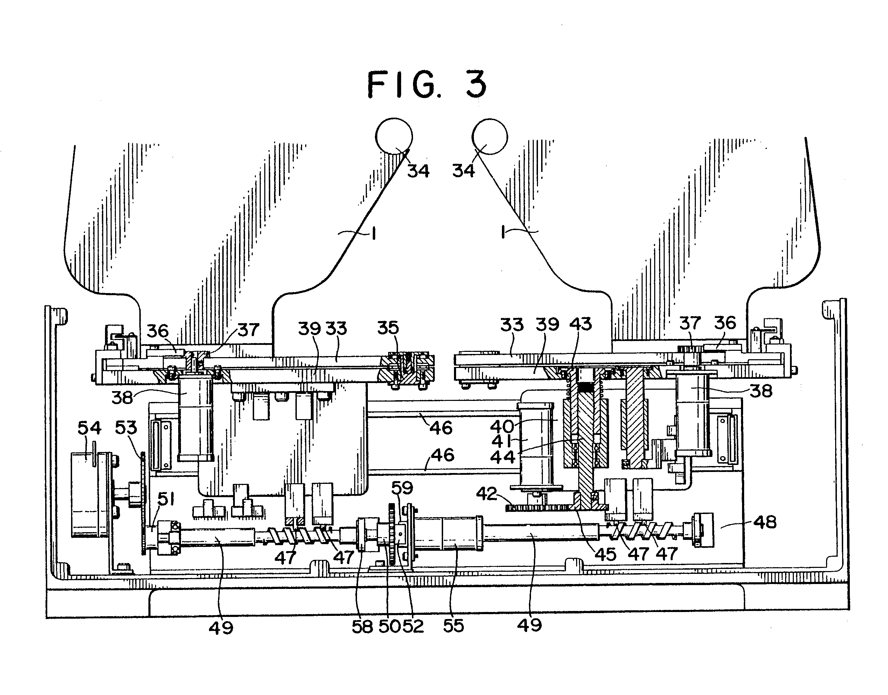

Figure 3 is a front view of a means for convergence of the setting plate 1, a means for raising and lowering one eye head i.e., a setting plate 1, and a means for adjusting pupillary distance. All such means are assembled in a casing which is fixed on a desk. The casing has two horizontal setting boards 39 for left and right eyes, both of which are slidably mounted on the casing by a means described hereinafter. Each of the boards 39 has, at each opposite end, a shaft 35, with a convergence plate 33 rotatably mounted on one end thereof, so that the convergence plate 33 can be rotated about the axis defined by the shaft 35. Each of the convergence plates 33 has the setting plate 1 which is screwed in place vertically, parallel to the plate 33, at the other end thereof. The setting plate 1 has a window 34 for measuring vision data whose center is on the center line of the shaft 35. The convergence plate 33 also has a gear 36 fixed to the end carrying the setting plate 1, the gear 36 engaging with a gear 37 attached to a shaft of a direct current motor (DC-motor) 38 fixed to the setting board 39. Rotary motion of both convergence plates 33, i.e., convergence of both setting plates 1, is produced by rotation of both DC-motors 38. Each of the boards 39 also has a sliding plate 40 which is fixed thereto and slides on a rail 46 fixed to a base 48 by sliding bearings. Each of the sliding plates 40 has two actuating pins 47 which are fixed at the bottom portion of 40 and are moved in both directions by the rotation of a leading screw 49, so that each sliding plate 40 slides on rail 46 by rotation of the leading screw 49, which is set by flanges 58 at the sides of the base 48 fixed to the casing, and both leading screws 49 are joined together by a flexible coupling joint 59. Attached to the one leading screw 49 are a gear 50 which engages with a gear 52 of a DC-motor 55, and a gear 51 which engages with a gear 53 attached to a shaft of a sensor 54 for showing pupillary distance. The sliding plate 40 for the left eye, shown on the right side in Figure 3, has a mechanism for raising and lowering one eye head, in which a gear 42 attached to a DC-motor 41 fixed to sliding plate 40 engages with a gear 45 attached to a vertical screw 44 engaged with a screw thread on the inside the wall of a cylinder 43 fixed vertically to the setting board 39, so that a rising and falling motion of cylinder 43 i.e., a rising and falling motion of setting board 39 or setting plate 1, is produced by rotation of DC-motor 41. Figure 3 does not show bolts and pins for attaching various assemblies of the apparatus.

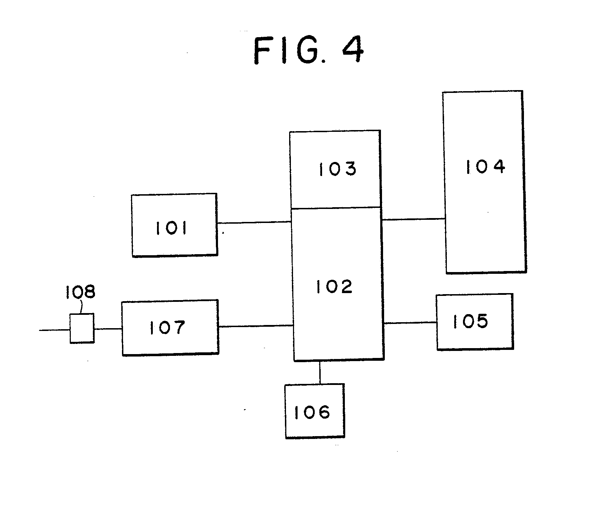

Figure 4 is a schematic block diagram showing the elements of an apparatus of this invention. In Figure 4, 101 is a control switch board, 102 is a basic board for input and output, 103 is a basic board for a central processing unit (hereinafter referred to as the CPU), 104 is a unit for measuring vision data, 105 is a monitor, 106 is a printer, 107 is an electric power source unit, and 108 is a breaker switch. The measuring unit 104 has eight pulse motors, four DC-motors, twelve zero position sensors, and four electro-magnetic clutches. Operation of the actuating means can be controlled via the input-output board 102 and the CPU board 103 by control keys or switches 101 on the desk, so that various kinds of vision data of patients can be obtained simply by operation of the switches, and all the data obtained can be digitally displayed in monitor 105 on the desk, and if necessary, can be printed out by printer 106 on the desk.

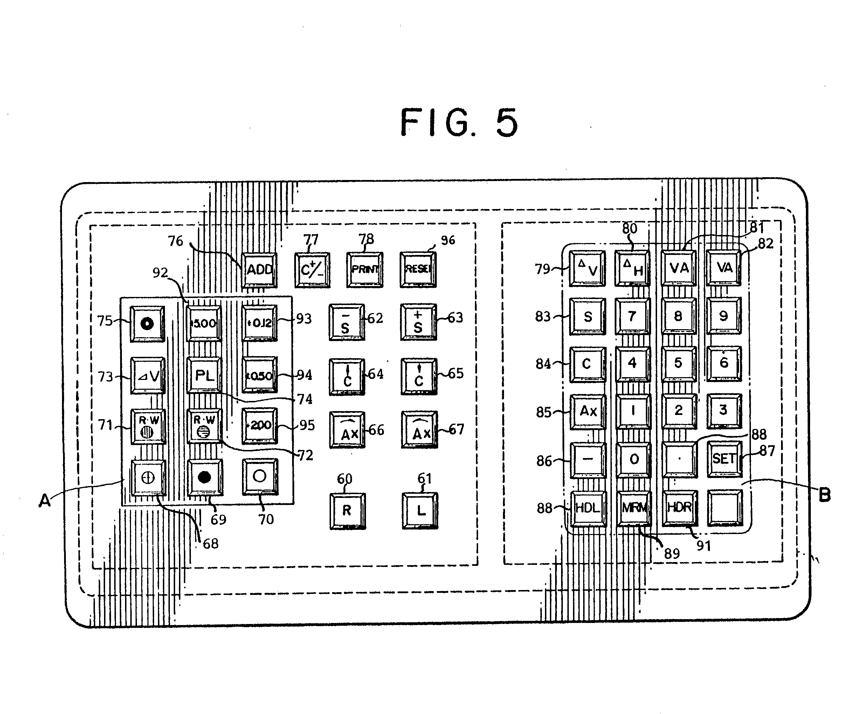

Figure 5 is a plan view of a control switch board. Figure 5 does not show the key or switch for raising and lowering one eye head and the key or switch for adjusting pupillary distance that are associated with the measuring unit 104, but the switches of course may be disposed in the board of Figure 5. 60 and 61 are switches for deciding whether the control switches are to be applied to either the right-eye head or the left-eye head. 62 and 63 are switches for rotating either clockwise or counterclockwise the pulse motor 9 connected to the spherical lens plate 2. Input from the switch 62 or 63 gives a signal to the input-output board 102, while a definite number of pulses are simultaneously sent to the input-output board 102 from CPU board 103 in which a micro-computer is disposed, so that the pulse motor 9 is driven. Pushing the switch 62 or 63 once makes lens plate 2 rotate by the minimum diopter (0.25 d) pitch, and continuing depression of the respective switch or key makes lens plate 2 continue to rotate at a constant speed until actuation of the key or switch is ended. At the position of just before one revolution of lens plate 2 is completed during the switch actuation operation, current is applied to the electro-magnetic clutch 11, so that lens plate 2 and lens plate 3 are rotated simultaneously, and the current is cut off after one pitch revolution of lens plates 2 and 3. The electro-magnetic clutch 11 has a double shaft assembly, either shaft of which is freely rotated independently when the current is cut off, while the double shafts are rotated together like one shaft when current is introduced.

Switch 64 or 65 is the one for rotating the toric lens plates. The motion which is produced by actuating these switches is almost the same as the motion in the case of the switches 62 and 63, but the toric lens can only have a cylindrical power of minus diopter, so that the arrow signs on the switches are employed. Simultaneous rotation of toric lens plate 15 and 16 is produced by the electro-magnetic clutch 20, as in the case of the spherical lens plates 2 and 3. Actuating switch 66 or 67 sends a number of pulses to the pulse motor 28, rotating the cylinder axes of all toric lenses of the toric lens plates 15 and 16 simultaneously in the same direction. The motion produced by actuating the switch is the same as with the switches 62 and 63, in that one pitch rotation corresponds to one actuation of the key or switch, and continued actuation produces continuing rotation at a constant speed until the actuation of the switch is ended.

Pushing a key or switch in the block A in Figure 5 causes the CPU board 103 to judge the number of pulses required, sending a signal to pulse motor 10, and rotating lecos plate 4. Each key or switch in the block A corresponds to each function which is included in an ordinary lecos plate so that 68 is a switch representing a cross-line for measuring P.D. which is used in setting pupillary distance. 69 or 70 is a switch for closing or opening the measuring window 34. 71 or 72 is a switch for Mardox which is used for measuring heterophoria. 95 is a switch for retinoscopy which is used for measuring retinoscopy objectively at a distance of 50 cm. 73 is a switch for a supplementary prism, and 74 is a switch for polaroid lenses, used for binocular testing. 94 is a switch for a fixed crossed-cylinder test for measuring hyperopia which is used for measuring additional diopters for close work. 75 is a switch for pin hole which is used for checking amblyopia. 92 is a switch for supplementary spherical lenses, used for measuring spherical power of up to +20 D. 93 is a switch for supplementary spherical lenses for intermediate power. 76 is a switch which is used for additional diopters necessary for close work, and is actuated to supply direct current to the DC-motor 38 for convergence, converging the convergence plates inward by a necessary angle, simultaneously supplying DC-voltage to the DC-motor 55 for measuring pupillary distance, and making the heads move a few millimeter inward from the pupillary distance for far work. 77 is a switch for C sign transposition, and 78 is a switch for printing vision data.

Switches in block B in Figure 5 are supplementary ones which are used for inputting previously measured data. 79 and 80 are switches used for inputting vertical prism power or horizontal prism power. 81 and 82 are used for inputting vision power without glasses, and vision power with glasses. 83, 84, 85, 86, the numeric switches (0)—(9), 87 and 88 are switches which are used to rotate lens plates in order to set appropriate lenses in front of a patient's eyes when vision data of the patient's eye glasses are previously known. For example, if S=1.00 D, C=0.50 D, and Ax=100°, switches are pushed in the order:

83 86 (1) 88 (0) (0) 84 86 (0)
　　　　　88 (5) (0) 85 (1) (0) (0) 87.

Electric power inputted by actuating these switches is applied to the input-output board 102, and then to the CPU board 103, for calculation of the number of pulses to be sent to pulse motor 9 in order to rotate the spherical lens plate, the number of pulses to be sent to the pulse motor 23 in order to rotate the toric lens plate, and the number of pulses to be sent to the pulse motor 28 in order to rotate the cylinder axes; the numbers of pulses calculated are applied to the respective pulse motors for setting each the lens plates and the cylinder axes. Switches 89, 90 and 91 are for inputting data measured by other instruments such as a lensmeter, an objective phorometer, etc. other than the subjective phorometer of this invention. If a certain one of these switches is pushed, the data is inputted to the CPU board 103, so that the number of pulses required is calculated, and sent to the pulse motor, and the lens plates and the cylinder axes are thus set.

In an example of this invention, disposed in the measuring case or enclosure is a key or switch for giving a DC-voltage to the DC-motor 41 for raising and lowering one eye head, and the switch for giving DC-voltage to the DC-motor 55 for adjusting pupillary distance, moving two heads in an inward or outward direction. Those switches can of course be included in the switch board of Fig. 5.

All the data such as data from each lens plate controlled by main switches of the switch board mentioned above, data inputted from supplementary switches of block B, and data from other instruments, can be displayed digitally on a monitor on the desk. The data in the monitor can be printed out by a high speed printer, if the switch 78 is pushed. After measuring is finished, the 96 switch is pushed, so that all the measuring means return to their zero positions in order to be ready to the next measurement operation.

**Claims**

1. A motor-driven subjective phorometer which comprises a right side and a left side mounting means (1) for a set of spherical and toric lens plates and an additional plate (4), the spherical lens plates including a first spherical lens plate (2) with a series of spherical lenses each having a different spherical power varying in small dioptral steps and a second spherical lens plate (3) with a series of spherical lenses each having a different spherical power varying in large dioptral steps, said toric lens plates including a first toric lens plate (15) with a series of toric lenses each having a different cylindrical power varying in small dioptral steps and a second toric lens plate (16) with a series of toric lenses each having a different cylindrical power varying in large dioptral steps, means for changing the relative position of said right side and left side mounting means (1), motor means (9, 10, 23, 28, 38, 55, 41) for controlling the motion of said lens and additional plates (2, 3, 4, 15, 16) and of said relative position changing means, control switching means (101, 102) operable through a central processing unit (103) to effect controlled actuation of said motor means, and means for digitally displaying measured data corresponding to the position of said motor means, characterized in that said mounting means are constituted by a right side and a left side plate (1), that the first spherical lens plate (2), the first toric lens plate (15) and the additional plate (4) are controlled by means of pulse motors (9, 10, 23, 28) and gear means (7, 8; 19, 21, 22), that the second spherical lens plate (3) and the second toric lens plate (16) are rotated together with the first spherical lens plate (2) and the first toric lens plate (15), respectively, through electro-magnetic clutch means (11, 20) having a double shaft mechanism, that raising and lowering motions of one side plate (1) on which the lens plates (2, 3) are disposed, convergence of the right side plate and the left side plate on which the lens plates are disposed and inward and outward sliding motion of the right side plate and the left side plate are obtained through direct current motor means (38, 55, 41) and gears (36, 37; 51, 53; 43, 44, 45), and that printing means are further provided for printing out said data as required.

2. A phorometer according to claim 1, wherein said control switching means include a central group of control switches (101).

3. A phorometer according to claim 2 wherein said central group of control switches comprises depressable keys or push-buttons.

4. A phorometer according to claim 1 or claim 2 and including means (13, 14; 29, 31; 30, 32) for detecting a given position of a respective lens plate (2, 3; 15, 16).

5. A phorometer according to any preceding claim, which is in the form of a desk-top apparatus.

**Patentansprüche**

1. Motorisch-betriebenes Sehprüfgerät umfassend eine rechtsseitige und eine linksseitige Halterung (1) für einen Satz sphärischer und torischer Linsenplatten und eine zusätzliche Platte (4), wobei die sphärischen Linsenplatten eine erste sphärische Linsenplatte (2) mit einer Reihe sphärischer Linsen umfassen, von denen jede eine andere sphärische Brechkraft hat, die sich in kleinen Dioptrieschritten ändert, und eine zweite sphärische Linsenplatte (3) mit einer Reihe sphärischer Linsen umfassen, von denen jede eine andere sphärische Brechkraft hat, die sich in großen Dioptrieschritten ändert, wobei die torischen Linsenplatten eine erste torische Linsenplatte (15) mit einer Reihe torischer Linsen umfassen, von denen jede eine andere zylindrische Brechkraft hat, die sich in kleinen Dioptrieschritten ändert, und eine zweite torische Linsenplatte (16) mit einer Reihe torischer Linsen umfassen, von denen jede eine andere zylindrische Brechkraft hat, die sich in großen Dioptrieschritten ändert, eine Einrichtung zur Änderung der relativen Lage von rechtsseitiger und linksseitiger Halterung (1), eine Motoranordnung (9, 10, 23, 28, 38, 55, 41) zur Steuerung der Bewegung der Linsenplatten und der zusätzlichen Platte (2, 3, 4, 15, 16) und der die relative Lage ändernden Einrichtung, eine Steuerschaltereinrichtung (101, 102), die von einer zentralen Verarbeitungseinheit (103) betätigbar ist, um einen gesteuerten Betrieb der Motoranordnung zu bewirken, und eine Einrichtung zur digitalen Anzeige der gemessenen Daten entsprechend der Stellung der Motoranordnung, dadurch gekennzeichnet, daß die Halterungen von einer rechtsseitigen und einer linksseitigen Platte (1) gebildet werden, daß die erste sphärische Linsenplatte (2), die erste torische Linsenplatte (15) und die zusätzliche Platte (4) mittels Schrittmotoren (9, 10, 23, 28) und einer Getriebeanordnung (7, 8; 19, 21, 22) gesteuert werden, daß die zweite sphärische Linsenplatte (3) und die zweite torische Linsenplatte (16) zusammen mit der ersten sphärischen Linsenplatte (2) bzw. der ersten torischen Linsenplatte (15) mittels elektromagnetischer Kupplungseinrichtungen (11, 20) gedreht werden, die einen Doppelwellenmechanismus aufweisen, daß die Hebe- und Senkbewegung einer Seitenplatte (1), an der die Linsenplatten (2, 3) angeordnet sind, die Konvergenz von rechter Seitenplatte und linker Seitenplatte, an der die Linsenplatten angeordnet sind, und die inwärts und auswärts gerichtete Verschiebebewegung der rechten Seitenplatte und der linken Seitenplatte mittels Gleichstrommotoranordnungen (38, 55, 41) und Getrieben (36, 37; 51, 53; 43, 44, 45) erzielt werden, und daß ferner eine Druckeinrichtung vorgesehen ist, um die Daten bedarfsweise auszudrucken.

2. Sehprüfgerät nach Anspruch 1, bei dem die Steuerschaltereinrichtung eine zentrale Gruppe von Steuerschaltern (101) aufweist.

3. Sehprüfgerat nach Anspruch 2, bei dem die zentrale Gruppe von Steuerschaltern Drucktasten oder Druckknöpfe umfaßt.

4. Sehprüfgerat nach Anspruch 1 oder 2, das Mittel (13, 14; 29, 31; 30, 32) zum Erfassen einer gegebenen Lage einer jeweiligen Linsenplatte (2, 3; 15, 16) aufweist.

5. Sehprüfgerät nach einem der vorhergehenden Ansprüche, das in Form eines Tischgeräts ausgebildet ist.

**Revendications**

1. Un dispositif motorisé d'évaluation subjective de l'acuité visuelle comprenant des moyens de montage du côté droit et des moyens de montage du côté gauche (1) pour un ensemble de plaques de support de lentilles sphériques et toriques et une plaque supplémentaire (4), les plaques de support de lentilles sphériques comprenant une première plaque de support de lentilles sphériques (2) portant une série de lentilles sphériques ayant chacune une vergence sphérique différente, variant par petits incréments de vergence et une seconde plaque de support de lentilles sphériques (3) portant une série de lentilles sphériques ayant chacune une vergence sphérique différente variant par grands incréments de vergence, les plaques de support de lentilles toriques comprenant une première plaque de support de lentilles toriques (15) portant une série de lentilles toriques ayant chacune une vergence cylindrique différente variant par petits incréments de vergence et une seconde plaque de support de lentilles toriques (16) portant une série de lentilles toriques ayant chacune une vergence cylindrique différente variant par grands incréments de vergence, des moyens destinés à changer la position relative des moyens de montage du côté droit et du côté gauche (1) des moyens à moteurs (9, 10, 23, 28, 38, 55, 41) destinés à commander le mouvement des plaques de support de lentilles et de la plaque supplémentaire (2, 3, 4, 15, 16) et des moyens de changement de position relative, des moyens de commutation de commande (101, 102) manoeuvrables par l'intermédiaire d'une unité centrale de traitement (103) pour actionner de façon commandée les moyens à moteurs, et des moyens destinés à afficher de façon numérique des données mesurées correspondant à la position des moyens à moteurs, caractérisé en ce que les moyens de montage sont constitués par une plaque latérale du côté droit et une plaque latérale du côté gauche (1), en ce que la première plaque de support de lentilles sphériques (2), la première plaque de support de lentilles toriques (15) et la plaque supplémentaire (4) sont commandées au moyen de moteurs à impulsions (9, 10, 23, 28) et d'engrenages (7, 8; 19, 21, 22), en ce que la seconde plaque de support de lentilles sphériques (3) et la seconde plaque de support de lentilles toriques (16) sont mises en rotation respectivement conjointement à la première plaque de support de lentilles sphériques (2) et à la première plaque de support de lentilles toriques (15), par l'intermédiaire d'embrayages électromagnétiques (11, 20) ayant un mécanisme à deux arbres, en ce que des mouvements de montée et de descente d'une plaque latérale (1) sur laquelle sont placées les plaques de support de lentilles (2, 3), la convergence de la plaque latérale droite et de la plaque latérale gauche sur lesquelles sont placées les plaques de support de lentilles, et le mouvement glissant vers l'intérieur et l'extérieur de la plaque latérale droite et de la plaque latérale gauche sont obtenus par des moyens à moteurs à courant continu (38, 55, 41) et par des engrenages (36, 37; 51, 53; 43, 44, 45), et en ce que des moyens d'impression sont en outre prévus pour imprimer les données précités lorsque c'est nécessaire.

2. Un dispositif d'évaluation de l'acuité visuelle selon la revendication 1, dans lequel les moyens de commutation de commande comprennent un groupe centrale de commutateurs de commande (101).

3. Un dispositif d'évaluation de l'acuité visuelle selon la revendication 2, dans lequel le groupe central de commutateurs de commande comprend des touches ou des boutons-poussoirs sur lesquels on peut appuyer.

4. Un dispositif d'évaluation de l'acuité visuelle selon la revendication 1 ou la revendication 2 et comprenant des moyens (13, 14: 29, 31; 30, 32) pour détecter une position donnée d'une plaque de support de lentilles respective (2, 3; 15, 16).

5. Un dispositif d'évaluation de l'acuité visuelle selon l'une quelconque des revendications précédentes, se présentant sous la forme d'un appareil à poser sur une table.

# FIG. 1(A)

# FIG. 1(B)

# FIG. 2(A)

# FIG. 2(B)

FIG. 3

# FIG. 4

# FIG. 5

0 098 442